# EUROPEAN PATENT SPECIFICATION

(11) **EP 1 453 838 B1**
(45) Date of publication and mention of the grant of the patent: **08.03.2006**
(21) Application number: 02804206.7
(22) Date of filing: 29.11.2002
(51) Int. Cl.: C07D 521/00

(54) **PRODUCTION OF IONIC LIQUIDS**
VERFAHREN ZUR HERSTELLUNG VON IONISCHEN FLÜSSIGKEITEN
PRODUCTION DE LIQUIDES IONIQUES

(30) Priority: 04.12.2001 EP 01128403
(43) Date of publication of application: 08.09.2004
(73) Proprietor: DSM IP Assets B.V., 6411 TE Heerlen (NL)
(72) Inventor: BONRATH, Werner, 79115 Freiburg (DE); LEVEQUE, Jean-Marc, Bâtiment "Le Solarium", F-73100 Aix-Les-Bains (FR); LUCHE, Jean-Louis, F-38120 Saint-Egreve (FR); PETRIER, Christian, F-73000 Chambery (FR)
(74) Representative: Schwander, Kuno
(86) International application number: PCT/EP2002/013499
(87) International publication number: WO 2003/048078

(56) References cited:
- JOAN FULLER ET AL.: "Structure of 1-Ethyl-3-methylimidazolium Hexafluorophosphate: Model for Room Temperature Molten Salts" J. CHEM. SOC. CHEM. COMMUN., 1994, pages 299-300, XP002240841
- WASSERSCHEID P ET AL: "Ionic Liquids - New Solutions for Transition Metal Catalysis" ANGEWANDTE CHEMIE. INTERNATIONAL EDITION, VERLAG CHEMIE. WEINHEIM, DE, vol. 39, no. 21, 27 October 2000 (2000-10-27), pages 3772,3778,3780-3787, XP002218385 ISSN: 0570-0833 cited in the application
- LEVEQUE, JEAN-MARC ET AL: "An improved preparation of ionic liquids by ultrasound" GREEN CHEMISTRY (2002), 4(4), 357-360 , XP008017060

## Description

The present invention relates to a process for the manufacture of ionic liquids by a technique not previously employed for this purpose.

The term "ionic liquid" denotes a salt that is a liquid at temperatures of about 100°C or below, i.e. the salt has a melting point of up to about 100°C, and consists only of ions. Ionic liquids belong to a known class of chemical compounds and are useful as solvents for organic syntheses. Ionic liquids have gained particular interest in view of their capability of replacing classical solvents which themselves can cause toxicological and disposal problems; by using ionic liquids instead of such classical solvents, these problems can be avoided.

Ionic liquids are known to be producible by anion exchange, e.g. by reacting a quaternary ammonium halide, featuring the cation component, with an acid or a salt thereof which provides the requisite anion component of the ionic liquid [see, for example, Chem. Rev. 99, 2071-2083 (1999) and Angew. Chem. Int. Ed. 39, 3772-3789 (2000)]. However, these known procedures are lengthy and generally result in reaction products which require purification. Due to the non-volatile nature of ionic liquids such purification cannot be accomplished by distillation but requires more elaborate procedures such as treatment with ion exchangers or extended treatment with charcoal.

In accordance with the present invention it has been found that that the production of such ionic liquids by anion exchange can advantageously be effected when carried out under ultrasonication. Compared with the prior art processes, the process of the present invention simplifies the manufacture of ionic liquids inasmuch as the process results in ionic liquids which can be isolated from the reaction mixture in satisfactory purity by filtration and removal of the solvent, if used.

Thus, the present invention is a process for the manufacture of ionic liquids by an anion exchange reaction of a quaternary ammonium, phosphonium, imidazolium or pyridinium halide, providing the appropriate cation component, with an acid or a metal salt thereof, providing the appropriate anion component, of the ionic liquid, wherein said reaction is carried out under ultrasonication.

Of particular interest for production by the process of the present invention are ionic liquids wherein the cation is a quaternary ammonium, phosphonium, imidazolium or pyridinium cation, for example tetraalkylammonium, e.g. tetra(n-butyl)-ammonium; tetraalkylphosphonium or phosphonium featuring both alkyl and aryl (particularly phenyl) groups, e.g. triisobutylmethylphosphonium, trihexyl-tetradecyl-phosphonium or triphenyl-octyl-phosphonium; N,N'-dialkyl-imidazolium, e.g. 1-ethyl-3-methylimidazolium, 1-(n-butyl)-3-methylimidazolium or 1-methyl-3-(n-propyl)-imidazolium; and N-alkyl-pyridinium or N,4-dialkyl-pyridinium, e.g. 1-(n-butyl)-pyridinium or 1-(n-butyl)-4-methylpyridinium, respectively. Where alkyl occurs in such cations this is generally alkyl containing 1 to 14 carbon atoms and which may be straight or branched chain.

Typical examples of anions of the ionic liquids produced by the process of the present invention are tetrafluoroborate (BF₄⁻), hexafluorophosphate (PF₆⁻), hexafluoroantimonate (SbF₆⁻), nitrate, bisulphate (hydrogen sulphate), tetraphenylborate [B(C₆H₅ )₄⁻], thiocyanate, acetate, hexyltriethylborate, trifluoromethylsulphonyl, nonafluorobutanesulphonate, bis[(trifluoromethyl)sulphonyl]imide, tris[(trifluoromethyl)sulphonyl]methide, trifluoroacetate and heptafluorobutanate, as well as anions based on chlorides and other halides of aluminum, copper, manganese, lead, cobalt, nickel or gold, e.g. tetrachloroaluminate (AlCl₄⁻), heptachlorodialuminate (Al₂Cl₇⁻) and tetrachlorocuprate (CuCl₄²⁻ and CuCl₄³⁻).

Any combination of one of the above-mentioned cations with one of the above-mentioned anions gives an example of an ionic liquid which can be produced by the process of the present invention. Such examples are 1-ethyl-3-methylimidazolium tetrachloroaluminate, 1-(n-butyl)-pyridinium nitrate, tetra(n-butyl)-ammonium acetate and 1-ethyl-3-methylimidazolium hexafluorophosphate.

The anion exchange reaction itself (not involving the inventive ultrasonication aspect) is known in the art and is described in several publications, including review articles, e.g. Polyhedron 15, No. 7, 1217-1219 (1996); J. Chem. Tech. Biotechnol. 68, 351-356 (1997); Chemistry & Engineering News March 1998, pp. 32-37; Chem. Prod. and Proc. 1, 223-236 (1999); Chem. Rev. 99, 2071-2083 (1999); Angew. Chem. 112, 3926-3945 (2000) and its international edition Angew. Chem. Int. Ed. 39, 3772-3789 (2000); and earlier references mentioned in these articles. As also indicated above, ionic liquids are generally produced by reacting a halide, particularly chloride but also bromide or iodide, of the quaternary ammonium, phosphonium, imidazolium or pyridinium cation, with an acid featuring the anion component, or a group I metal, particularly an alkali metal or silver, or ammonium salt thereof. Examples of said acid or salt are fluoroboric acid (HBF₄), hexafluorophosphoric acid (HPF₆), ammonium hexafluorophosphate (NH₄⁺PF₆⁻), trifluoromethanesulphonic acid and Lewis acids supplying the aforementioned halides of boron, aluminum, copper etc., e.g. boron trifluoride and aluminum trichloride.

In a preferred aspect the present invention is concerned with the manufacture of ionic liquids wherein the cation is an imidazolium or pyridinium cation, particularly 1-ethyl-3-methylimidazolium, 1-(n-butyl)-3-methylimidazolium or 1-methyl-3-(n-propyl)-imidazolium, or, respectively, 1-(n-butyl)-pyridinium. The preferred anions of the ionic liquid are tetrafluoroborate, hexafluorophosphate and trifluoromethanesulphonate. Thus, the reaction preferably involves reacting an imidazolium or pyridinium halide, particularly a 1-ethyl-3-methylimidazolium, 1-(n-butyl)-3-methylimidazolium, 1-methyl-3-(n-propyl)-imidazolium or 1-(n-butyl)-pyridinium halide, with a tetrafluoroborate or hexafluorophosphate, e.g. ammonium, an alkali metal or silver tetrafluoroborate or hexafluorophosphate, or a trifluoromethanesulphonate, e.g. ammonium trifluoromethanesulphonate, under ultrasonication. The halide of the reactant providing the cation component is preferably the chloride, although the bromide or iodide may also be used. The alkali metal cation of the salt reactant (if not an acid) providing the anion component may be lithium, sodium, potassium or cesium; however, the cation of said salt reactant is preferably silver or ammonium.

Suitably, the process of the present invention is carried out in an anhydrous solvent medium under a dry atmosphere, i.e. with as much exclusion of moisture as possible. However, the use of a solvent is not essential. Suitable solvents are organic solvents which are inert to the reactants and the desired reaction product, i.e. the ionic liquid, and in which the ionic liquid is soluble. Suitable such solvents are aliphatic ketones, e.g. acetone and diethyl ketone, and aliphatic, alicyclic or aromatic hydrocarbons, e.g. heptane, cyclohexane or toluene, respectively. The employed solvent is suitably anhydrous to the extent of at least 99%, i.e. contains no more than 1% water (volume %). For the manufacture of ionic liquids wherein the cation is an imidazolium ion dry (anhydrous) acetone is preferably used as the reaction solvent.

The reaction is conveniently carried out at temperatures from about 0°C to about 40°C, preferably from about 10°C to about 30°C, and most preferably at about room temperature. Accordingly, the reaction is suitably carried out with cooling so as to absorb the excess heat produced by the ultrasonication.

The molar ratio of the employed reactants quaternary ammonium, phosphonium, imidazolium or pyridinium halide : acid or salt providing the required anion is conveniently about 1: 1 or up to about 1 : 1.1, i.e. featuring up to an about 10% molar excess of the acid or salt. In respect of the amount of solvent, if used, this is such that the ratio of solvent to the aforementioned halide reactant is conveniently from about 0.5 to about 10 ml of solvent: 1 mmol of the halide reactant.

The ultrasonication can be carried out using conventional equipment for ultrasonication in general at a frequency of at least about 20 kHz, preferably at frequencies from about 20 kHz to about 100 kHz, most preferably from about 30 kHz to about 50 kHz. The acoustic power output, although not narrowly critical, should be such as to exceed the cavitation threshold in the reaction mixture or medium. A power input of about 150 W to about 300 W has been found to be convenient. An example of an ultrasonifier (ultrasound apparatus) which may be employed is the Branson Model 250/450 Sonifier®, available from Branson Ultrasonics Corp., Eagle Roar, Danbury, CT 06810-1961, USA.

The progress of the ion exchange formation of the ionic liquid can be monitored, e.g. by measuring the electrical conductivity of the reaction mixture which, in general, changes during the ion exchange reaction and reaches a plateau on completion of the reaction. Another option to determine the progress of the ion exchange formation of the ionic liquid is by infrared spectroscopy. Such analytical methods are conventionally used in observing the progress of chemical reactions. Once the ion exchange reaction is complete the ionic liquid may be isolated by filtering off the halide formed in the reaction and evaporating the solvent, both being conventional isolation methods.

The following Examples illustrate the present invention.

### Example 1

To 5.23 g (30 mmol) of 1-(n-butyl)-3-methylimidazolium chloride [prepared from 1-methylimidazole and n-butane chloride in toluene as described in Polyhedron, 15,1217-1219 (1996)] and 30 ml of dry acetone there were added 30 mmol of ammonium tetrafluoroborate under an argon atmosphere in an ultrasonic reactor (Branson Model 250 Sonifier®) equipped with a cooling jacket, a cryostat, an argon balloon and an inlet tube for introducing the reaction solution and for taking samples to a conductimetric cell for measuring the conductivity throughout the experiment. The conductivity was measured with a Tacussel CDRV 62 conductimeter (Tacussel electronique, 72, rue d'Alsace, F-69627 Villeurbanne, Cedex France). The resulting slurry was irradiated using an Ultrasons Annemasse generator (Ultrasons Annemasse S.A., F-74103 Annemasse, Cedex France) at 30 kHz for 1 hour at 20-24°C, the cooling bath temperature being 5°C. The conductivity of the reaction mixture increased during the reaction up to a value of 65 milliSiemens (mS). After 1 hour, no further substantial increase of the conductivity was observed, so that the reaction was taken to have gone to completion. The contents of the reactor were filtered, the solvent evaporated off under reduced pressure, and the produced ionic liquid, 1-(n-butyl)-3-methylimidazolium tetrafluoroborate, recovered in 80-90% yield as a pale yellow to orange coloured liquid.

In a comparative experiment using the same starting materials but wherein the reaction mixture was stirred instead of being ultrasonicated a reaction time of 30 hours was required to obtain a 80-90 % yield of the desired ionic liquid, the product in this case having a dark brown colour.

### Example 2

In analogy to Example 1 but using ammonium hexafluorophosphate as the reactant providing the anion for the ion exchange there was obtained 1-(n-butyl)-3-methylimidazolium hexafluorophosphate in 80-90% yield as a pale yellow to orange coloured liquid.

### Example 3

In analogy to Example 1 but using ammonium trifluoromethanesulphonate as the reactant providing the anion for the ion exchange there was obtained 1-(n-butyl)-3-methylimidazolium trifluoromethanesulphonate in 80-90% yield as a pale yellow to orange coloured liquid.

## Claims

1. A process for the manufacture of ionic liquids by an anion exchange reaction of a quaternary ammonium, phosphonium, imidazolium, or pyridinium halide, providing the appropriate cation component, with an acid or a salt thereof, providing the appropriate anion component, of the ionic liquid, wherein said reaction is carried out under ultrasonication.

2. A process according to claim 1, wherein the cation component of the halide is an imidazolium or pyridinium cation, preferably a 1-ethyl-3-methylimidazolium, 1-(n-butyl)-3-methylimidazolium or 1-methyl-3-(n-propyl)-imidazolium halide, or respectively, a 1-(n-butyl)-pyridinium halide.

3. A process according to claim 1 or claim 2, wherein the halide is the chloride, bromide or iodide, preferably the chloride.

4. A process according to any one of claims 1 to 3, wherein the anion component of the acid or the salt thereof is tetrafluoroborate, hexafluorophosphate or trifluoromethanesulphonate.

5. A process according to any one of claims 1 to 4, wherein the cation component of the acid or the salt thereof is ammonium, an alkali metal or silver, preferably ammonium or silver.

6. A process according to any one of claims 1 to 5, wherein the ultrasonication is effected at a frequency of at least about 20 kHz, preferably at frequencies from about 20 kHz to about 100 kHz, most preferably at frequencies from about 30 kHz to about 50 kHz.

7. A process according to any one of claims 1 to 6 wherein the reaction is carried out in an anhydrous solvent medium under a dry atmosphere.

8. A process according to claim 7, wherein the solvent is an organic solvent which is inert to the reactants and the desired reaction product and is an aliphatic ketone or an aliphatic, alicyclic or aromatic hydrocarbon.

9. A process according to claim 8, wherein the solvent is acetone, diethyl ketone, heptane, cyclohexane or toluene.

10. A process according to any one of claims 1 to 9, wherein the reaction is carried out at temperatures from about 0°C to about 40°C, preferably from about 10°C to about 30°C, most preferably at about room temperature.

11. A process according to any one of claims 1 to 10, wherein the molar ratio of the employed reactants quaternary ammonium, phosphonium, imidazolium or pyridinium halide : acid or salt is about 1 : 1 to about 1:1.1.

12. A process according to any one of claims 7 to 11, wherein the ratio of the solvent to the quaternary ammonium, phosphonium, imidazolium or pyridinium halide reactant is from about 0.5 to about 10 ml of solvent: 1 mmol of the halide reactant.

## Patentansprüche

1. Verfahren zur Herstellung von ionischen Flüssigkeiten durch eine Anionenaustauschreaktion eines quartären Ammonium-, Phosphonium-, Imidazolium- oder Pyridiniumhalogenids, das die geeignete Kationenkomponente der ionischen Flüssigkeit bereitstellt, mit einer Säure oder einem Metallsalz davon, das die geeignete Anionenkomponente der ionischen Flüssigkeit bereitstellt, wobei die Reaktion unter Ultraschallbehandlung durchgeführt wird.

2. Verfahren nach Anspruch 1, wobei die Kationenkomponente des Halogenids ein Imidazolium- oder Pyridiniumkation ist, vorzugsweise ein 1-Ethyl-3-methylimidazolium-, 1-(n-Butyl)-3-methylimidazolium- oder 1-Methyl-3-(n-propyl)-imidazoliumhalogenid oder ein 1-(n-Butyl)-pyridiniumhalogenid.

3. Verfahren nach Anspruch 1 oder Anspruch 2, wobei das Halogenid das Chlorid, Bromid oder Iodid, vorzugsweise das Chlorid ist.

4. Verfahren nach einem der Ansprüche 1 bis 3, wobei die Anionenkomponte der Säure oder des Salzes davon Tetrafluorborat, Hexafluorphosphat oder Trifluormethansulfonat ist.

5. Verfahren nach einem der Ansprüche 1 bis 4, wobei die Kationenkomponente der Säure oder des Salzes davon Ammonium, ein Alkalimetall oder Silber, vorzugsweise Ammonium oder Silber, ist.

6. Verfahren nach einem der Ansprüche 1 bis 5, wobei die Ultraschallbehandlung bei einer Frequenz von mindestens etwa 20 kHz, vorzugsweise bei Frequenzen von etwa 20 kHz bis etwa 100 kHz, am stärksten bevorzugt bei Frequenzen von etwa 30 kHz bis etwa 50 kHz durchgeführt wird.

7. Verfahren nach einem der Ansprüche 1 bis 6, wobei die Reaktion in einem wasserfreien Lösungsmittelmedium unter trockener Atmosphäre durchgeführt wird.

8. Verfahren nach Anspruch 7, wobei das Lösungsmittel ein organisches Lösungsmittel ist, das zu den Reaktanten und dem gewünschten Reaktionsprodukt inert ist, und ein aliphatisches Keton oder ein aliphatischer, alicyclischer oder aromatischer Kohlenwasserstoff ist.

9. Verfahren nach Anspruch 8, wobei das Lösungsmittel Aceton, Diethylketon, Heptan, Cyclohexan oder Toluol ist.

10. Verfahren nach einem der Ansprüche 1 bis 9, wobei die Reaktion bei Temperaturen von etwa 0 °C bis etwa 40 °C, vorzugsweise etwa 10 °C bis etwa 30 °C, am stärksten bevorzugt bei etwa Raumtemperatur durchgeführt wird.

11. Verfahren nach einem der Ansprüche 1 bis 10, wobei das Molverhältnis der eingesetzten Reaktanten, quartäres Ammonium-, Phosphonium-, Imidazolium- oder Pyridiniumhalogenid : Säure oder Salz, etwa 1 : 1 bis etwa 1 : 1,1 beträgt.

12. Verfahren nach einem der Ansprüche 7 bis 11, wobei das Verhältnis des Lösungsmittels zu dem quartären Ammonium-, Phosphonium-, Imidazolium- oder Pyridiniumhalogenidreaktanten etwa 0,5 bis etwa 10 ml Lösungsmittel : 1 mmol Halogenidreaktant beträgt.

## Revendications

1. Procédé de production de liquides ioniques par une réaction d'échange d'anions d'un halogénure d'ammonium quaternaire, de phosphonium, d'imidazolium ou de pyridinium, présentant le composant cationique approprié, avec un acide ou un sel de celui-ci, présentant le composant anionique approprié, du liquide ionique, dans lequel ladite réaction est réalisée sous ultra-sonification.

2. Procédé selon la revendication 1, dans lequel le composant cationique de l'halogénure est un cation d'imidazolium ou de pyridinium, de préférence un halogénure de 1-éthyl-3-méthylimidazolium, de 1-(n-butyl)-3-méthylimidazolium ou de 1-méthyl-3-(n-propyl)-imidazolium, ou respectivement, un halogénure de 1-(n-butyl)-pyridinium.

3. Procédé selon la revendication 1 ou la revendication 2, dans lequel l'halogénure est le chlorure, le bromure ou l'iodure, de préférence le chlorure.

4. Procédé selon l'une quelconque des revendications 1 à 3, dans lequel le composant anionique de l'acide ou du sel de celui-ci est le tétrafluoroborate, l'hexafluorophosphate ou le trifluorométhanesulfonate.

5. Procédé selon l'une quelconque des revendications 1 à 4, dans lequel le composant cationique de l'acide ou du sel de celui-ci est l'ammonium, un métal alcalin ou de l'argent, de préférence, l'ammonium ou l'argent.

6. Procédé selon l'une quelconque des revendications 1 à 5, dans lequel l'ultra-sonification est réalisée à une fréquence d'au moins environ 20 kHz, de préférence à des fréquences d'environ 20 kHz à environ 100 kHz, de manière préférée entre toutes à des fréquences d'environ 30 kHz à environ 50 kHz.

7. Procédé selon l'une quelconque des revendications 1 à 6, dans lequel la réaction est réalisée dans un milieu de solvant anhydre sous une atmosphère sèche.

8. Procédé selon la revendication 7, dans lequel le solvant est un solvant organique qui est inerte par rapport aux réactifs et le produit de réaction désiré et est une cétone aliphatiqué ou un hydrocarbure aliphatique, alicyclique ou aromatique.

9. Procédé selon la revendication 8, dans lequel le solvant est l'acétone, la cétone diéthylique, l'heptane, le cyclohexane ou le toluène.

10. Procédé selon l'une quelconque des revendications 1 à 9, dans lequel la réaction est réalisée à des températures d'environ 0°C à environ 40°C, de préférence d'environ 10°C à environ 30°C, de manière préférée entre toutes à température ambiante environ.

11. Procédé selon l'une quelconque des revendications 1 à 10, dans lequel le rapport molaire des réactifs utilisés halogénure d'ammonium quaternaire, de phosphonium, d'imidazolium ou de pyridinium:acide ou sel est d'environ 1:1 à environ 1:1,1.

12. Procédé selon l'une quelconque des revendications 7 à 11, dans lequel le rapport du solvant au réactif halogénure d' ammonium quaternaire, de phosphonium, d'imidazolium ou de pyridinium est d'environ 0,5 à environ 10 ml de solvant : 1 mmol du réactif halogénure.
